# EUROPÄISCHE PATENTANMELDUNG

(11) **EP 2 674 133 A1**
(43) Veröffentlichungstag der Anmeldung: **18.12.2013**
(21) Anmeldenummer: 12171967.8
(22) Anmeldetag: 14.06.2012
(51) Int. Cl.: A61F 2/44

(54) **Zwischenwirbelfusionsimplantat**

(71) Anmelder: WALDEMAR LINK GmbH & Co. KG, 22339 Hamburg (DE)
(72) Erfinder: Link, Helmut D., 22397 Hamburg (DE); Dmuschewsky, Klaus, 22397 Hamburg (DE)
(74) Vertreter: Glawe, Delfs, Moll

(57) **Zusammenfassung**

Die Erfindung betrifft ein Zwischenwirbelfusionsimplantat (1) zur Fusion zweier Wirbel (52, 53). Das Zwischenwirbelfusionsimplantat zur Fusion zweier Wirbel (52, 53) umfasst einen oberen Lagerkörper (2) zur Anlage an eine untere Endplatte eines Wirbels (52) und gegenüberliegend einen unteren Lagerkörper (3) zur Anlage an eine obere Endplatte eines Wirbels (53), sowie ein zwischen den Lagerkörpern (2, 3) angeordnetes Mittelstück (6), wobei das Mittelstück als eine Klauenkupplung (9) ausgebildet ist, die in einer Koppelstellung die beiden Lagerkörper (2, 3) drehfest gegeneinander koppelt, wobei in einer offenen Stellung der Klauenkupplung (9) die Lagerkörper (2, 3) gegeneinander frei beweglich sind, wobei die Klauenkupplung (9) automatisch betätigt ist und als Betätigungsorgan einen plastischen Sperrkörper (10) aufweist, der in seiner initialen Stellung die Klauenkupplung (9) offen hält und unter einer von dem einen Wirbel (52) auf den anderen Wirbel (54) übertragenen Lastkraft langsam verformbar ist bis zum Erreichen einer finalen Koppelstellung, in der die Klauenkupplung (9) geschlossen ist. Durch die langsame Fusion der Wirbel (52, 53) bemerkt der Patient die Bewegungseinschränkung seiner Wirbelsäule nicht und sein Bewegungsapparat kann sich an die Veränderung langsam gewöhnen.

## Beschreibung

Die Erfindung betrifft ein Zwischenwirbelfusionsimplantat zur Fusion zweier Wirbel umfassend einen oberen Lagerkörper zur Anlage an eine untere Endplatte eines Wirbels und einen unteren Lagerkörper zur Anlage an eine obere Endplatte eines gegenüberliegenden Wirbels, sowie ein zwischen den Lagerkörpern angeordnetes Mittelstück. Weiter betrifft die Erfindung ein System aus einem Zwischenwirbelfusionsimplantat und einer Zange und ein Verfahren zum Einsetzen des Zwischenwirbelfusionsimplantats.

Aufgrund von Verletzung, Verschleiß und Krankheit kann eine Bandscheibe degenerieren, wobei sie häufig Druck auf die Nervenstränge des Rückenmarks ausübt. Dies kann starke Schmerzen oder Taubheitsgefühle in den Extremitäten bis hin zu Lähmungserscheinungen zur Folge haben. Daher kann es nötig werden, die degenerierte Bandscheibe zu entfernen. Allerdings muss der Kontakt zwischen den Wirbeln weiterhin vermieden werden, da ansonsten durch die Reibung zwischen den Endplatten der Wirbel Schmerzen verursacht werden. Weiter können medizinische Gründe vorliegen, eine Relativbewegung der an der degenerierten Bandscheibe anliegenden Wirbel zukünftig zu vermeiden. Die degenerierte Bandscheibe wird in diesen Fällen durch ein Zwischenwirbelfusionsimplantat ersetzt, das eine Relativbewegung der Wirbel verhindert.

Bekannte Zwischenwirbelfusionsimplantate weisen Lagerkörper zur Anlage an die Endplatten eines oberen und unteren Wirbels auf. Sie werden entweder direkt zwischen die Endplatten zweier Wirbel eingesetzt oder in Rinnen, die in die Endplatten gebohrt wurden, wobei Knochensubstanz der Wirbel entfernt wird. Die bekannten Implantate fusionieren die zwei an ihnen anliegenden Wirbel direkt nach der Implantation. Nachteil dieser Implantate ist, dass der Patient sich plötzlich auf die eingeschränkte Bewegungsfähigkeit seiner Wirbelsäule einstellen muss. Zum Einen bemerkt er die Einschränkung durch das Implantat. Zum Anderen muss sich auch sein Bewegungsapparat sofort auf die neue Situation einstellen. So sind die Bänder und Muskeln der Wirbelsäule auch direkt nach der Operation noch zur Bewegung der beiden fusionierten Wirbel ausgebildet. Dies kann in der Zeit nach der Operation zu einer verlängerten Eingewöhnungsphase und Rehabilitation des Patienten führen. Weiter ist das Implantat in der Eingewöhnungsphase höheren mechanischen Belastungen unterworfen als nach der Eingewöhnung.

Aufgabe der Erfindung ist daher, die oben genannten Nachteile zu vermeiden.

Die erfindungsgemäße Lösung liegt in den Merkmalen der unabhängigen Ansprüche. Vorteilhafte Weiterbildungen sind Gegenstand der abhängigen Ansprüche.

Gemäß der Erfindung ist ein Zwischenwirbelfusionsimplantat zur Fusion zweier Wirbel vorgesehen, das einen oberen Lagerkörper zur Anlage an eine untere Endplatte eines Wirbels und einen unteren Lagerkörper zur Anlage an eine obere Endplatte eines gegenüberliegenden Wirbels, sowie ein zwischen den Lagerkörpern angeordnetes Mittelstück umfasst, wobei das Mittelstück als eine Klauenkupplung ausgebildet ist, die in einer Koppelstellung die beiden Lagerkörper drehfest gegeneinander koppelt, wobei in einer offenen Stellung der Klauenkupplung die Lagerkörper gegeneinander frei beweglich sind, wobei die Klauenkupplung automatisch betätigt ist und als Betätigungsorgan einen plastischen Sperrkörper aufweist, der in seiner initialen Stellung die Klauenkupplung offen hält und unter einer von dem einen Wirbel auf den anderen Wirbel übertragenen Lastkraft langsam verformbar ist bis zum Erreichen einer finalen Koppelstellung, in der die Klauenkupplung geschlossen ist.

Nachfolgend seien zunächst einige Begriffe näher erläutert:

Unter einer Klauenkupplung wird eine Vorrichtung verstanden, die in einer offenen Stellung eine Drehbewegung der Lagerkörper gegeneinander erlaubt und in einer Koppelstellung die Lagerkörper drehfest gegeneinander koppelt. Es ist es nicht nötig, dass die Klauenkupplung eine Vielzahl von Fortsätzen umfasst. Sie kann auch lediglich einen Fortsatz umfassen, der mit einer Ausnehmung an der Klauenkupplung zusammenwirken kann. Die Ausgestaltung der Fortsätze kann an die gewünschte anfängliche, übergangsweise und finale Beweglichkeit der Lagerkörper angepasst werden. Dabei können die Fortsätze beispielsweise als Klauen, Zähne oder zylindrische oder konische Stifte ausgeführt sein. Wesentlich ist, dass die am oberen und unteren Lagerkörper angeordneten Fortsätze formschlüssig miteinander in Eingriff gebracht werden können.

Unter einer initialen offenen Stellung wird eine expandierte Anordnung der Klauenkupplung verstanden, die eine Bewegung der Lagerkörper gegeneinander zulässt. Der Sperrkörper ist in der offenen Stellung nicht verformt, so dass die Fortsätze der Klauenkupplung nicht ineinander eingreifen und eine freie Rotation der Lagerkörper gegeneinander ermöglichen. Weiter hat das Implantat in der offenen Stellung seine maximale Höhe.

Unter einer finalen Koppelstellung wird eine komprimierte Anordnung der oberen und unteren Lagerkörper sowie des Mittelstücks verstanden, in der die Lagerkörper aneinander gekoppelt sind. Die Klauenkupplung ist in der finalen Koppelstellung geschlossen. Trotz der geschlossenen Klauenkupplung kann gegebenenfalls eine Restbeweglichkeit zwischen den Lagerkörpern verbleiben. Das Implantat hat in der Koppelstellung seine minimale Höhe.

Unter einer langsamen plastischen Verformung wird eine irreversible Änderung der Form eines Körpers verstanden, die in einer Zeitspanne geschieht, die länger als eine Stunde andauert und vorzugsweise höchstens 24 Monate. Eine langsame Verformung weist demnach eine große Zeitkonstante auf.

Unter einer freien Bewegung der Lagerkörper wird eine Bewegung verstanden, die lediglich durch den Bandapparat der Wirbelsäule bzw. andere anatomische Gegebenheiten begrenzt ist, jedoch nicht durch die Klauenkupplung.

Kern der Erfindung ist, die Klauenkupplung des Implantats mittels einer langsamen plastischen Verformung des Sperrkörpers von einer offenen Stellung in eine Koppelstellung zu überführen. Durch die initiale offene Stellung des Implantats kann ein Patient seine Wirbelsäule in den Tagen nach der Implantation ohne Einschränkungen drehen. Der Sperrkörper ist in der Bewegungsstellung nicht verformt, so dass die Fortsätze der Klauenkupplung nicht ineinander eingreifen. Daher können die durch das Implantat verbundenen Wirbel gegeneinander gedreht werden. Wegen der Lastbeaufschlagung des Implantats in der Wirbelsäule des Patienten wird eine Druckkraft auf den Sperrkörper ausgeübt. Diese Kraft verformt den Sperrkörper plastisch, wobei der Sperrkörper so ausgestaltet ist, dass die plastische Verformung langsam bewirkt wird. Die Verformung des Sperrkörpers bewirkt eine allmähliche Überführung des Implantats von der offenen in die Koppelstellung und eine Kompression des Imlantats. Während der Überführung überlappen die offene und die Koppelstellung. In dieser Überlappphase ist der Sperrkörper noch nicht vollständig verformt und bewirkt eine teilweise Schließung der Klauenkupplung. Die Fortsätze der Klauenkupplung greifen in dieser Phase nicht vollständig ineinander ein und können somit um einen begrenzten Winkel gegeneinander verdreht werden. Daher können sich die Lagerkörper und die an ihnen anliegenden Wirbel in der Überlappphase nicht mehr frei gegeneinander drehen. Eine Drehung ist lediglich bis zu einem maximalen Drehwinkel noch möglich. Eine stärkere Verformung des Sperrkörpers bewirkt eine Verkleinerung des maximalen Drehwinkels. Das bedeutet, dass die Beweglichkeit des Implantats kontinuierlich abnimmt, bis schließlich die Koppelstellung erreicht ist.

In der Koppelstellung sind die Fortsätze der Klauenkupplung so angeordnet, dass sie ineinander eingreifen. Daher ist in der Koppelstellung die Klauenkupplung geschlossen, wobei die durch das Implantat verbundenen Wirbel gekoppelt werden. Es sei angemerkt, dass gegebenenfalls eine geringe Restbeweglichkeit in der Koppelstellung verbleiben kann. Weiter ist es nicht zwingend, dass Lagerkörper und Mittelstück gesonderte Teile sind. Sie können auch ineinander übergehen.

Auf Grund des langsamen Übergangs von der offenen in die Koppelstellung ist der Patient damit nicht mit einer plötzlichen Einschränkung der Bewegungsfreiheit seiner Wirbelsäule konfrontiert. Die Fusion der Wirbel fällt dem Patienten daher nicht deutlich auf. Weiter hat der Bewegungsapparat des Patienten auf Grund der langsamen Einschränkung der Bewegungsfähigkeit in der Überlappphase genügend Zeit, sich an die neue Situation zu gewöhnen. Damit sinkt die Belastung auf den Bewegungsapparat, die fusionierten Wirbel und das Implantat.

Mit Vorteil weist die Klauenkopplung Fortsätze auf, die am oberen und unteren Lagerstück angeordnet sind und zum jeweils anderen Lagerstück weisen.

Vorteilhafterweise ist der Sperrkörper in das Mittelstück integriert, wobei der Sperrkörper vorzugsweise modular austauschbar und/oder vorzugsweise als Scheibe ausgestaltet ist. Damit kann mittels der Stärke des Sperrkörpers die Zeitkonstante der Verformung einfach eingestellt werden. Die Fortsätze der Klauenkupplung können dabei auf dem Sperrkörper gleitend angeordnet sein. Weiter bewirkt die Verformung des Sperrkörpers, dass der Weg, den die Fortsätze auf dem Sperrkörper gleiten können, begrenzt wird. Durch die Verformung greifen die Fortsätze ineinander ein. Sie prägen einem als Scheibe ausgestalteten Sperrkörper durch die Lastkraft der Wirbelsäule eine Wellenform auf. Die Fortsätze müssen daher in diesem Fall bei einer Rotation der Lagerkörper auf einer gewellten Scheibe gleiten, wodurch der Weg, den sie auf planer Fläche zurücklegen können, verkleinert wird.

Weiter sind die Fortsätze in der Koppelstellung mit Vorteil zur Selbstzentrierung und Rotationsbremsung ausgebildet, wobei der plastisch verformte Sperrkörper als Rotationsbremse fungiert. Vorzugsweise sind die Fortsätze dazu abgerundet. Ein abgerundeter Fortsatz führt den Teil des sich verformenden Sperrkörpers, der die Bewegung des Fortsatzes begrenzt, in eine abgerundete Form über und vermeidet Beschädigungen. Weiter wirkt der verformte Sperrkörper über den abgerundeten Fortsatz als eine sich stufenlos verstärkende Rotationsbremse. Die abgerundeten Fortsätze gleiten dabei in dem abgerundeten Teil des Sperrkörpers wie in einer Wanne, wodurch sie zentriert werden. An den Grenzen der Wanne bewirkt die Gleitbewegung der Fortsätze eine Öffnung der Klauenkupplung. Das Implantat muss damit für eine Drehung, die die Fortsätze über die Begrenzung führt, expandiert werden. Dies wird durch die Bänder und die Last der Wirbelsäule erschwert, die so als weicher Anschlag wirken.

In einer ersten bevorzugten Ausführungsform weist das Zwischenwirbelfusionsimplantat einen axialen am Sperrkörper angeordneten oberen Stift, der in einer Axialbohrung des oberen Lagerkörpers angeordnet ist, und einen axialen am Sperrkörper angeordneten unteren Stift auf, der in einer Axialbohrung des unteren Lagerkörpers angeordnet ist. Die Stifte wirken in Kombination mit den Axialbohrungen als Innenführung für den Sperrkörper. Sie zentrieren den Sperrkörper zwischen den Lagerkörpern und verhindern, dass der Sperrkörper von den Lagerkörpern weg gedrückt wird.

In einer zweiten bevorzugten Ausführungsform weist das Zwischenwirbelfusionsimplantat eine Auffangwanne auf, die vorzugsweise als um die Klauenkupplung laufende Umgürtung ausgebildet ist. Die Auffangwanne fungiert weiter als Außenführung, die den Sperrkörper zwischen den Lagerkörpern zentriert. Weiter fängt die Auffangwanne Abrieb auf, der durch die initiale Bewegungsfähigkeit der Klauenkupplung entsteht, so dass er nicht in das umliegende Gewebe einwandern kann. Damit werden Komplikationen nach der Implantation vermindert.

In einer dritten bevorzugten Ausführungsform umfasst das Zwischenwirbelfusionsimplantat eine obere und eine untere radial am Sperrkörper umlaufende Rinne, wobei die Fortsätze in der Rinne angeordnet sind. In dieser Ausführungsform fungiert die Rinne als Führung für die Fortsätze und als Zentrierhilfe für den Sperrkörper.

Vorteilhafterweise sind der obere und untere Lagerkörper in einer vierten bevorzugten Ausführungsform identisch ausgebildet. Damit wird eine kostengünstige Produktion ermöglicht und der Zusammenbau des Implantats vereinfacht.

In einer fünften bevorzugten Ausführungsform weist der obere Lagerkörper mit Vorteil ein oberes Haltemodul und der untere Lagerkörper ein unteres Haltemodul auf.

Es ist zweckmäßig, wenn die Klauenkupplung an jedem Lagerkörper vier oder mehr Fortsätze aufweist. Damit wird eine hohe Stabilität des Implantats in Bezug auf Kippbewegungen der Wirbelsäule erreicht.

Mit Vorteil weisen die Lagerkörper Lagerflächen mit einer knochenwachstums-fördernden Beschichtung auf. Die Beschichtung bewirkt ein schnelles Einwachsen der Wirbel mit den an ihnen angeordneten Lagerkörpern. Damit wird die Stabilität des Implantats und der finalen Fusion erhöht.

Weiter weist das Zwischenwirbelfusionsimplantat mit Vorteil in der Koppelstellung eine Höhe zwischen 3 mm und 9 mm, vorzugsweise 6 mm, und in der offenen Stellung eine Höhe zwischen 9,5 mm und 20 mm, vorzugsweise 12,5 mm, und einen Durchmesser zwischen 12 mm und 30 mm auf, wobei der Sperrkörper eine Stärke zwischen 0,5 mm und 3 mm aufweist. Unter der Stärke des Sperrkörpers wird die räumliche Ausdehnung entlang der Drehachse der Klauenkupplung verstanden.

Zweckmäßigerweise besteht der Sperrkörper aus einem gleitgünstigen zähfesten Kunststoff, insbesondere PEEK oder UHMWPE.

Weiter bestehen die Lagerkörper und die Fortsätze zweckmäßigerweise aus einer metallischen biokompatiblen Legierung, insbesondere aus Reintitan, einer Titanlegierung, einer Kobaltbasislegierung CoCr oder CoCrMo; oder aus einem hochfesten biokompatiblen Kunststoff, insbesondere PEEK, PEEK-Kohlenstoff oder Keramik.

Die Erfindung betrifft ferner ein System aus einem Zwischenwirbelfusionsimplantat zur Fusion zweier Wirbel umfassend einen oberen Lagerkörper zur Anlage an eine untere Endplatte eines Wirbels und einen unteren Lagerkörper zur Anlage an eine obere Endplatte eines gegenüberliegenden Wirbels, sowie ein zwischen den Lagerkörpern angeordnetes Mittelstück, und einer Zange mit einem Maul, das ein erstes und ein zweites Backenelement aufweist, wobei das Mittelstück als eine Klauenkupplung ausgebildet ist, die in einer Koppelstellung die beiden Lagerkörper drehfest gegeneinander koppelt, wobei in einer offenen Stellung der Klauenkupplung die Lagerkörper gegeneinander frei beweglich sind, wobei die Klauenkupplung automatisch betätigt ist und als Betätigungsorgan einen plastischen Sperrkörper aufweist, der in seiner initialen Stellung die Klauenkupplung offen hält und unter einer von dem einen Wirbel auf den anderen Wirbel übertragenen Lastkraft langsam verformbar ist bis zum Erreichen einer finalen Koppelstellung, in der die Klauenkupplung geschlossen ist; und die Backenelemente zur positionsversetzten Anordnung der am oberen und unteren Lagerkörper angeordneten Fortsätze ausgebildet ist, so dass die Fortsätze des oberen Lagerkörpers neben den Fortsätzen des unteren Lagerkörpers angeordnet sind.

Durch die positionsversetzte Anordnung sind die Fortsätze in einer Zahn-auf-Lücke-Anordnung positioniert, wenn die Wirbelsäule keiner Verdrehung unterliegt. Damit ist sichergestellt, dass das Implantat die an ihm anliegenden Wirbel in einem unverdrehten Zustand fusioniert.

Mit Vorteil weist die Zange ein Anschlagselement zur Einstellung eines Mindestabstands der Backenelemente auf, wobei die Backenelemente das Zwischenwirbelfusionsimplantat in der offenen Stellung aufnehmen. Damit wird verhindert, dass der Sperrkörper bereits vor der Implantation durch die Kraftwirkung der Zange verformt wird.

In Bezug auf Weiterbildungen des Zwischenwirbelfusionsimplantats wird auf die vorhergehende Beschreibung verwiesen. Die Erfindung betrifft weiter ein Verfahren zur Fusion zweier Wirbelkörper mittels eines Zwischenwirbelfusionsimplantats zur Fusion zweier Wirbel, das einen oberen Lagerkörper zur Anlage an eine untere Endplatte eines Wirbels und gegenüberliegend einen unteren Lagerkörper zur Anlage an eine obere Endplatte eines Wirbels, sowie ein zwischen den Lagerkörpern angeordnetes Mittelstück umfasst, wobei das Mittelstück als eine Klauenkupplung ausgebildet ist, die in einer Koppelstellung die beiden Lagerkörper drehfest gegeneinander koppelt, wobei in einer offenen Stellung der Klauenkupplung die Lagerkörper gegeneinander frei beweglich sind, wobei die Klauenkupplung automatisch betätigt ist und als Betätigungsorgan einen plastischen Sperrkörper aufweist, der in seiner initialen Stellung die Klauenkupplung offen hält und unter einer von dem einen Wirbel auf den anderen Wirbel übertragenen Lastkraft langsam verformbar ist bis zum Erreichen einer finalen Koppelstellung, in der die Klauenkupplung geschlossen ist;
umfassend folgende Schritte:
Vorbereitung einer Implantationsstelle;
Einsetzen des Zwischenwirbelfusionsimplantats in der expandierten offenen Stellung in die Implantationsstelle; und
Verschließen der Implantationsstelle.

Zweckmäßigerweise wird das Einsetzen des Zwischenwirbelfusionsimplantats mittels einer Zange ausgeführt, die ein Maul umfasst, das ein erstes und ein zweites Backenelement aufweist, wobei die Backenelemente zur positionsversetzten Anordnung der Fortsätze ausgebildet sind, so dass die Fortsätze des oberen Lagerkörpers neben den Fortsätzen des unteren Lagerkörpers angeordnet sind.

Vorteilhafterweise ist die Implantationsstelle zur Aufnahme des Zwischenwirbelfusionsimplantats in der expandierten offenen Stellung ausgebildet.

In Bezug auf Weiterbildungen des Zwischenwirbelfusionsimplantats und der Zange wird auf die vorhergehende Beschreibung verwiesen.

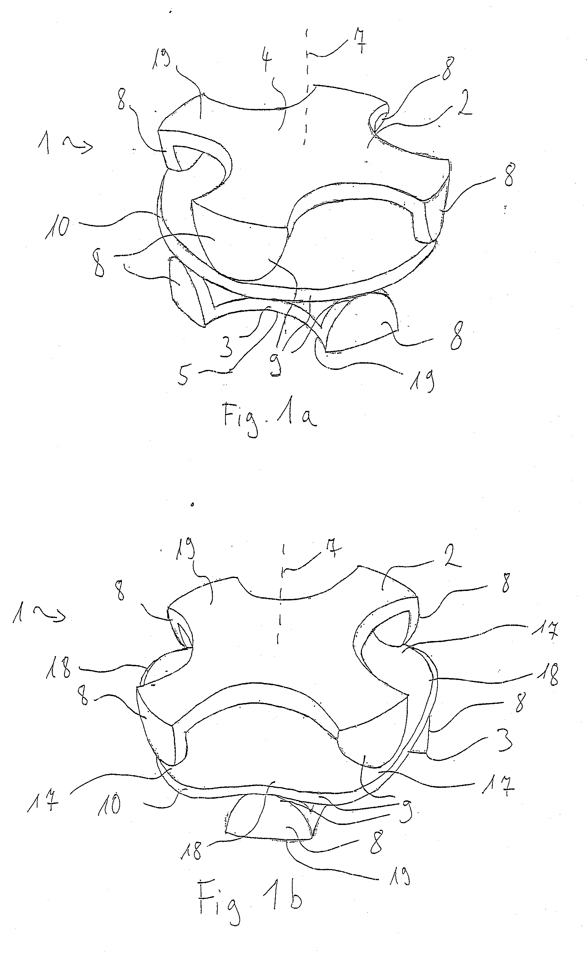

Nachfolgend wird die Erfindung mit Bezug auf die beigefügte Zeichnung, die eine vorteilhafte Ausführungsform zeigt, näher erläutert. Es zeigen
- Fig. 1a, b: eine schematische Darstellung eines Zwischenwirbelfusionsimplantats mit unverformten (a) und verformtem (b) integrierten Sperrkörper;
- Fig. 2a-c: schematische Darstellungen eines Zwischenwirbelfusionsimplantats mit Innenführung (a), Außenführung (b) und Rinne (c);

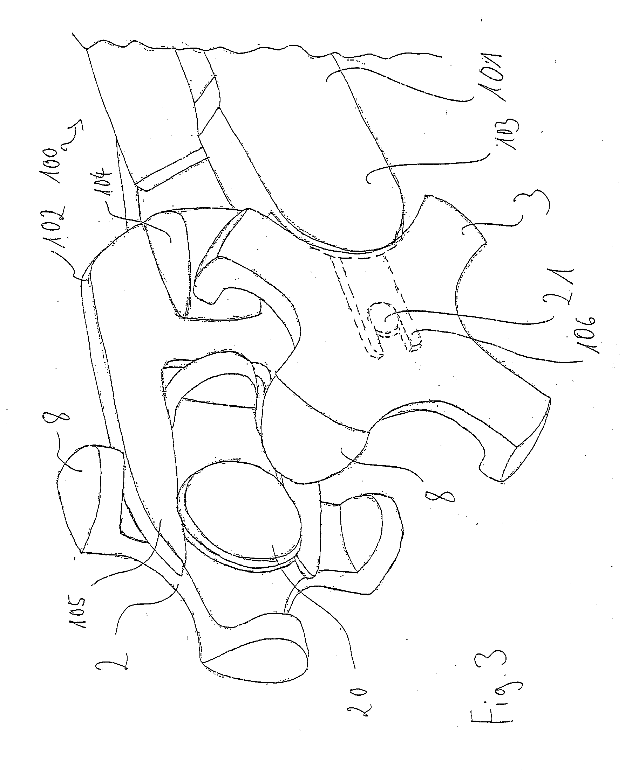

- Fig. 3: eine schematische Darstellung einer Zange mit gegriffenen Lagerkörpern;

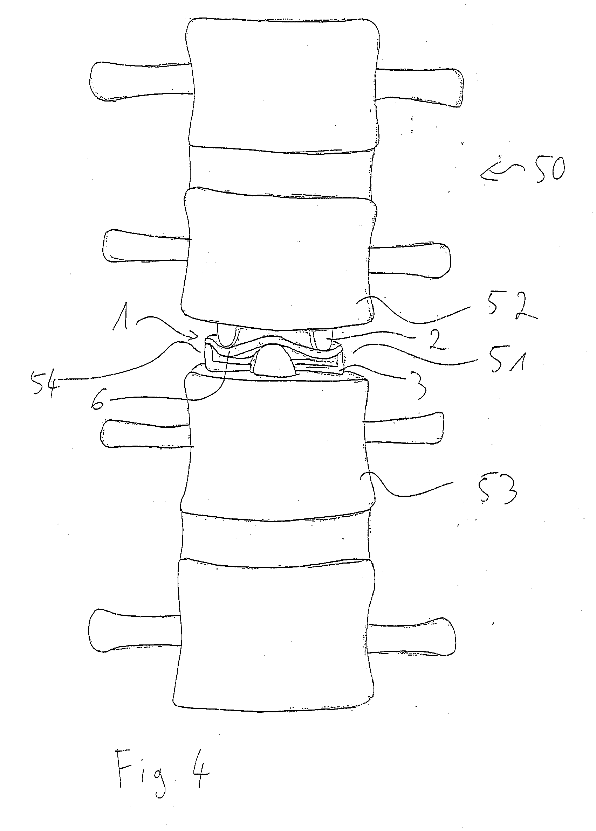

- Fig. 4: eine schematische Darstellung eines Teilstücks einer Wirbelsäule mit eingesetztem Zwischenwirbelfusionsimplantat; und
- Fig. 5a-c: eine schematische Darstellung des Zwischenwirbelfusionsimplantats in verschiedenen Ausführungsformen.

Das Zwischenwirbelfusionsimplantat wird in seiner Gesamtheit mit der Bezugsziffer 1 bezeichnet. Es weist einen oberen Lagerkörper 2, einen unteren Lagerkörper 3 und eine zwischen den Lagerkörpern 2, 3 angeordnete Klauenkupplung 9 auf. Die Lagerkörper 2, 3 sind dazu ausgebildet an die untere bzw. obere Deckplatte eines Wirbels 52, 53 angeordnet zu werden. Dazu weisen sie Lagerflächen 4, 5 auf. Quer zu den Deckplatten der Wirbel 52, 53 ist eine Drehachse 7 der Klauenkupplung 9 angeordnet. Weiter sind an den Lagerkörpern 2, 3 jeweils vier Fortsätze 8 der Klauenkupplung 9 angeordnet, die zum jeweils anderen Lagerkörper 2, 3 weisen. Die Fortsätze 8 sind abgerundet. Weiter sind sie gleitend auf einem Sperrkörper 10 angeordnet, so dass sie eine Rotation um die Drehachse 7 ausführen können. Mittels der Klauenkopplung 9 können die Lagerkörper 2, 3 damit in der offenen Stellung gegeneinander um die Drehachse 7 verdreht werden. In die Klauenkupplung 9 ist ein modular austauschbarer Sperrkörper 10 integriert, der langsam plastisch verformt werden kann.

Das Zwischenwirbelfusionsimplantat 1 wird in der initialen expandierten offenen Stellung in einen Zwischenwirbelraum 51 eines Patienten eingesetzt. In der Bewegungsstellung sind die Fortsätze 8 des oberen Lagerkörpers 2 auf Lücke zu den Fortsätzen 8 des unteren Lagerkörpers 3 angeordnet. Auf das Zwischenwirbelfusionsimplantat 1 wirkt im Zwischenwirbelraum 51 durch die Last, die die Wirbelsäule 50 trägt, eine Druckkraft. Die Druckkraft wird von dem oberen Lagerkörper 2 auf die an ihm angeordneten Fortsätze 8 übertragen. Über den Kontakt der Fortsätze 8 mit dem Sperrkörper 10 wirken die Druckkräfte von oben auf den Sperrkörper 10 ein. Durch die versetzte Positionierung der Fortsätze 8 des oberen und unteren Lagerkörpers 2, 3 weist der Sperrkörper 10 Druckpositionen 17 auf, an denen die am oberen Lagerkörper 2 angeordneten Fortsätze 8 auf den Sperrkörper 10 eine Kraft ausüben. Weiter weist der Sperrkörper 10 Stützpositionen 18 auf, an denen die am unteren Lagerkörper 3 angeordneten Fortsätze 8 den Sperrkörper 10 abstützen. An den Druckpositionen 17 wird der Sperrkörper 10 langsam plastisch verformt, indem die Fortsätze 8 relativ zu den Stützpositionen 18 eine Vertiefung in den Sperrkörper 10 drücken.

Sobald die plastische Verformung beginnt, wird das Zwischenwirbelfusionsimplantat 1 komprimiert. Um die Fortsätze 8 des oberen Lagerkörpers 2 quer zu einer Vertiefung in Richtung einer Stützposition 18 zu bewegen, muss das Zwischenwirbelfusionsimplantat 1 expandiert werden. Damit muss für diese Bewegung der Zwischenwirbelraum 51 durch den Patienten vergrößert werden. Eine Vergrößerung des Zwischenwirbelraums 51 wird allerdings durch die an der Wirbelsäule 50 angeordneten Bänder (nicht dargestellt) sowie durch die auf der Wirbelsäule 50 liegende Last erschwert. Der Zwischenwirbelraum 51 muss immer weiter vergrößert werden, je weiter sich ein am oberen Lagerkörper 2 angeordneter Fortsatz 8 einer Stützposition 18 nähert. Damit vergrößert sich auch der Widerstand, den der Patient bei einer Drehung zu überwinden hat, so dass der Sperrkörper 10 als eine sich stufenlos verstärkende Rotationsbremse auf die Klauenkupplung 9 wirkt. Das gleiche gilt analog für eine Bewegung der Fortsätze 8 des unteren Lagerkörpers 3 in Richtung einer Druckposition 17.

Sobald die Vertiefungen so ausgeprägt sind, dass keine Bewegung der Fortsätze 8 mehr möglich ist, befindet sich das Zwischenwirbelfusionsimplantat 1 in der Koppelstellung. Die Klauenkupplung 9 ist in der Koppelstellung vollständig geschlossen. Die Fortsätze 8 des oberen und unteren Lagerkörpers 2, 3 greifen in der Koppelstellung ineinander ein, so dass ein Fortsatz 8 des oberen Lagerkörpers 2 zwischen zwei Fortsätzen 8 des unteren Lagerkörpers 3 angeordnet ist und umgekehrt. Eine Bewegung der an den Lagerkörpern 2, 3 anliegenden Wirbel 52, 53 gegeneinander um die Drehachse 7 ist nur bis zu einer geringen Restbeweglichkeit möglich. Die Wirbel 52, 53 sind damit fusioniert.

Zur Fixierung des Zwischenwirbelfus.ionsimplantats 1 an die Wirbel 52, 53 können die Lagerkörper 2, 3 eine Substanz aufweisen die das Knochenwachstum fördert. Dazu wird auf den Lagerflächen 4, 5 die Substanz als Beschichtung 19 aufgetragen. Mittels der Beschichtung 19 verbinden sich die Lagerkörper 2, 3 mit den Endplatten der Wirbel 52, 53 und werden an ihrer Position fixiert. Die Gefahr eines Verrutschens des Zwischenwirbelfusionsimplantats 1 wird damit minimiert.

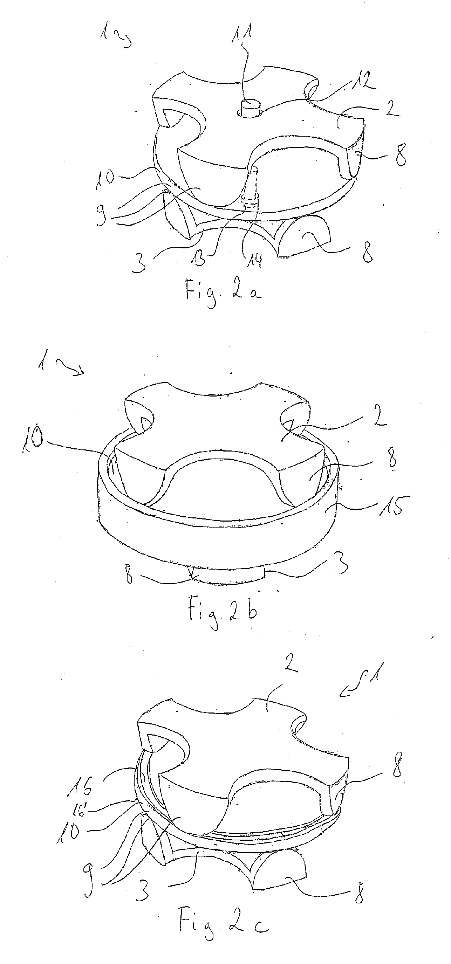

In Fig. 2a wird eine erste Ausführungsform des Zwischenwirbelfusionsimplantats 1 dargestellt. Die Lagerkörper 2, 3 weisen axiale Bohrungen 12, 14 auf, in denen ein oberer und ein unterer vom Sperrkörper 10 abragender Stift 11, 13 angeordnet sind. Die Bohrungen 12, 14 bilden eine Führung für die Stifte 11, 13, so dass der Sperrkörper 10 keine Bewegung in radialer Richtung durchführen kann. Die Stifte 11, 13 bestehen wie der Sperrkörper 10 aus einem plastisch verformbaren Material. Mittels der Stifte 11, 13 wird der Sperrkörper 10 zwischen den Lagerkörpern 2, 3 zentriert und die Lagerkörper 2, 3 relativ zum Sperrkörper 10 drehbar gelagert.

In einer in Fig. 2b dargestellten zweiten Ausführungsform weist das Zwischenwirbelfusionsimplantat 1 eine Auffangwanne 15 auf, die kragenförmig um den Sperrkörper 10 angeordnet. ist. Die Auffangwanne 15 ist als Umgürtung ausgeführt. Die Auffangwanne 15 bildet für die auf dem Sperrkörper 10 angeordneten Fortsätze 8 eine Begrenzung in radialer Richtung. Damit verhindert die Auffangwanne 15, dass der Sperrkörper 10 von den Lagerkörper 2, 3 in radialer Richtung weg gedrückt wird. Weiter fängt die Auffangwanne 15 Abrieb, der bei einer Bewegung des Zwischenwirbelfusionsimplantats 1 durch die Reibung zwischen den Fortsätzen 8 und dem Sperrkörper 10 entsteht, auf. Damit kann der Abrieb nicht in das umliegende Gewebe einwandern und dort Komplikationen wie, z. B. Entzündungen verursachen.

Eine dritte in Fig. 2c dargestellte Ausführungsform des Zwischenwirbelfusionsimplantat 1 weist auf dem Sperrkörper 10 angeordnete Rinnen 16, 16' auf, in denen die Enden der Fortsätze 8 angeordnet sind. Die Rinnen 16, 16' bilden eine Führung für die Fortsätze 8 und verhindern ein Verrutschen des Sperrkörpers 10 von den Lagerkörpern 2, 3.

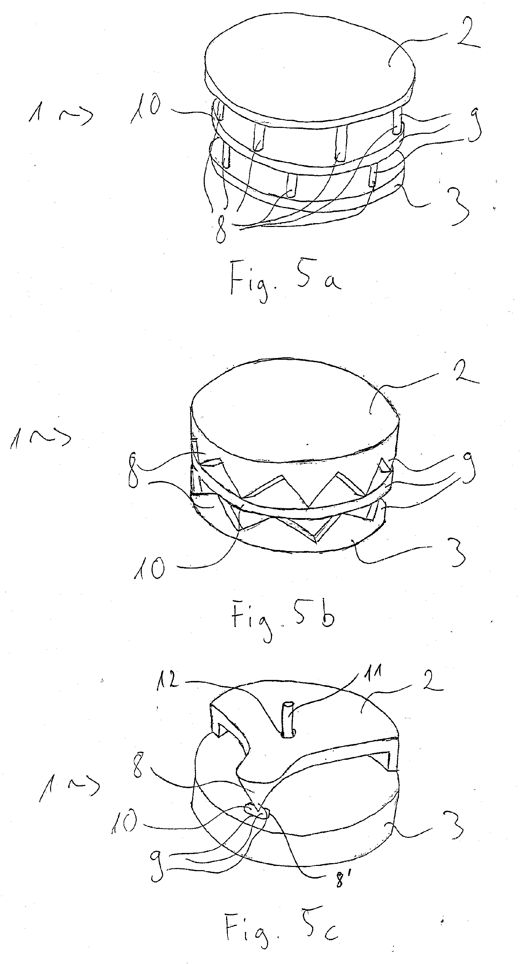

In den Figuren 5a bis 5c werden weitere Ausführungsformen des Zwischenwirbelimplantats 1 dargestellt, bei der die Fortsätze 8 nicht wie vorstehend beschrieben klauenartig geformt sind. Die Fortsätze 8 sind in der in Fig. 5a als zylindrische Stifte und in Fig. 5b als Zahnradzähne ausgebildet. Fig. 5c zeigt eine Ausführungsform des Zwischenwirbelimplantats 1, in der die Klauenkupplung 9 einen einzelnen als konischen Pin ausgebildeten Fortsatz 8 am oberen Lagerkörper 2 aufweist. Zwischen dem Fortsatz 8 und dem unteren Lagerkörper 3 ist der Sperrkörper 10 in einer Ausnehmung 8' des unteren Lagerkörpers 3 angeordnet. Durch die Lastkraft der Wirbelsäule 50 verformt der Fortsatz 8 den Sperrkörper 10 langsam plastisch. Durch die Verformung des Sperrkörpers 10 sinkt der Fortsatz 8 in die Ausnehmung 8' ein und die Klauenkupplung 9 wird allmählich geschlossen. Damit werden der obere und der untere Lagerkörper gekoppelt.

Der Sperrkörper 10 besteht aus UHMWPE. UHMWPE weist eine glatte Oberfläche auf und ist mit den obenangegebenen Maßen langsam plastisch verformbar. Weiter bestehen die Lagerkörper 2, 3 und die Fortsätze 8 aus einer Titanlegierung. Titanlegierungen sind biokompatibel und weisen im Körper eines Patienten eine sehr gute Korrosionsbeständigkeit auf.

Das Zwischenwirbelfusionsimplantat 1 weist in der Koppelstellung eine Höhe zwischen 3 mm und 9 mm, vorzugsweise 6 mm, auf. In der offenen Stellung weist es eine Höhe zwischen 9,5 mm und 20 mm, vorzugsweise 12,5 mm, auf. Dabei weist der Sperrkörper 10 eine Stärke zwischen 0,5 mm und 3 mm auf. Der Durchmesser des Zwischenwirbelfusionsimplantats 1 beträgt zwischen 12 mm und 30 mm. Mittels der Stärke des Sperrkörpers 10 mit dem integrierten Sperrkörper 10 wird die Zeitkonstante der langsamen plastischen Verformung festgelegt. Je stärker der Sperrkörper 10 ausgebildet ist, desto größer ist die Zeitkonstante der Verformung.

Um in den Zwischenwirbelraum 51 eines Patienten eingeführt zu werden, kann das Zwischenwirbelfusionsimplantat 1 mit einer Zange 100 gegriffen werden. Dazu kann der obere Lagerkörper 2 ein oberes Haltemodul 20 und der untere Lagerkörper 3 ein unteres Haltemodul 21 aufweisen. Die Zange 100 weist ein Maul 101 auf, das ein erstes und ein zweites Backenelement 102, 103 umfasst. Das erste Backenelement 102 weist ein erstes Greifmodul 105 auf, das so komplementär zu einem Fortsatz 8 ausgebildet ist, dass es einen Fortsatz 8 aus radialer Richtung umgreifen kann. Weiter ist das erste Greifmodul 105 zum Greifen des oberen Haltemoduls 20 ausgebildet. Das zweite Backenelement 103 weist ein zweites Greifmodul 106 auf. Das zweite Greifmodul 106 ist dazu ausgebildet radial zwischen zwei Fortsätze 8 des unteren Lagerkörpers 3 einzugreifen und das untere Haltemodul 21 zu greifen. Die Fortsätze 8 eines Lagerkörpers 2, 3, der von dem ersten Backenelement 102 gegriffen wird, und die Fortsätze 8 eines Lagerkörpers 2, 3, der von dem zweiten Backenelement 103 gegriffen wird, sind daher zueinander positionsversetzt um die Drehachse 7 angeordnet. Die Fortsätze 8 eines Lagerkörpers 2, 3 weisen dabei zwischen die Fortsätze 8 des gegenüberliegenden Lagerkörpers 2, 3. Wenn das Zwischenwirbelfusionsimpl.antat 1 mittels der Zange 100 gehalten wird, ist es in der offenen Stellung.

Weiter umfasst die Zange 100 ein Anschlagselement 104, das einen Mindestabstand zwischen den Backenelementen 102, 103 einstellt. Das Anschlagselement 104 kann im Maul 101 der Zange 100 angeordnet sein. Mit dem Anschlagselement 104 wird verhindert, dass durch den Druck der Zange 100 auf das Zwischenwirbelfusionsimplantat 1 der Sperrkörper 10 verformt wird.

Die Implantation des Zwischenwirbelfusionsimplantats 1 erfolgt in einem ersten Schritt durch die Vorbereitung einer Implantationsstelle 54 am Patienten. Der Chirurg legt den Zwischenwirbelraum 51 mit der defekten Bandscheibe frei und entfernt die Bandscheibe. Danach wird der Zwischenwirbelraum 51 gegebenenfalls so weit erweitert, dass das Zwischenwirbelfusionsimplantat 1 in der expandierten offenen Stellung in ihn eingesetzt werden kann. Die Erweiterung kann mittels Auseinanderziehen der Wirbel 52, 53 oder durch Extraktion von Knochensubstanz bewirkt werden. Das Zwischenwirbelfusionsimplantats 1 wird mit der Zange 100 in der offenen Stellung in den Zwischenwirbelraum 51 eingesetzt. Danach wird die Implantationsstelle 54 verschlossen.

## Patentansprüche

1. Zwischenwirbelfusionsimplantat zur Fusion zweier Wirbel (52, 53) umfassend einen oberen Lagerkörper (2) zur Anlage an eine untere Endplatte eines Wirbels (52) und einen unteren Lagerkörper (3) zur Anlage an eine obere Endplatte eines gegenüberliegenden Wirbels (53), sowie ein zwischen den Lagerkörpern (2, 3) angeordnetes Mittelstück,
**dadurch gekennzeichnet, dass**
das Mittelstück als eine Klauenkupplung (9) ausgebildet ist, die in einer Koppelstellung die beiden Lagerkörper (2, 3) drehfest gegeneinander koppelt, wobei in einer offenen Stellung der Klauenkupplung (9) die Lagerkörper (2, 3) gegeneinander frei beweglich sind, wobei die Klauenkupplung (9) automatisch betätigt ist und als Betätigungsorgan einen plastischen Sperrkörper (10) aufweist, der in seiner initialen Stellung die Klauenkupplung (9) offen hält und unter einer von dem einen Wirbel (52) auf den anderen Wirbel (53) übertragenen Lastkraft langsam verformbar ist bis zum Erreichen einer finalen Koppelstellung, in der die Klauenkupplung (9) geschlossen ist.

2. Zwischenwirbelfusionsimplantat nach Anspruch 1,
**dadurch gekennzeichnet, dass**
die Klauenkupplung (9) Fortsätze (8) aufweist, die am oberen und unteren Lagerstück (2, 3) angeordnet sind und zum jeweils anderen Lagerstück (2, 3) weisen.

3. Zwischenwirbelfusionsimplantat nach einem der Ansprüche 1 oder 2,
**dadurch gekennzeichnet, dass**
der Sperrkörper (10) in das Mittelstück integriert ist, wobei der Sperrkörper (10) vorzugsweise modular austauschbar ist und/oder vorzugsweise als Scheibe ausgestaltet ist.

4. Zwischenwirbelfusionsimplantat nach einem der Ansprüchen 1 bis 3,
**dadurch gekennzeichnet, dass**
die Fortsätze (8) in der Koppelstellung zur Selbstzentrierung und Rotationsbremsung ausgebildet sind, wobei der plastisch verformte Sperrkörper (10) als Rotationsbremse fungiert, wobei die Fortsätze (8) vorzugsweise abgerundet sind.

5. Zwischenwirbelfusionsimplantat nach einem der vorhergehenden Ansprüche,
**dadurch gekennzeichnet, dass**
das Zwischenwirbelfusionsimplantat (1) einen axialen am Sperrkörper (10) angeordneten oberen Stift (11), der in einer Axialbohrung (12) des oberen Lagerkörpers (2) angeordnet ist, und einen axialen am Mittelstück (6) angeordneten unteren Stift (13) aufweist, der in einer Axialbohrung (14) des unteren Lagerkörpers (3) angeordnet ist.

6. Zwischenwirbelfusionsimplantat nach einem der Ansprüche 1 bis 4,
**dadurch gekennzeichnet, dass**
das Zwischenwirbelfusionsimplantat (1) eine Auffangwanne (15) aufweist, die vorzugsweise als um die Klauenkupplung (9) laufende Umgürtung ausgebildet ist.

7. Zwischenwirbelfusionsimplantat nach einem der Ansprüche 1 bis 4,
**dadurch gekennzeichnet, dass**
das Zwischenwirbelfusionsimplantat (1) eine obere und eine untere radial am Mittelstück (6) umlaufende Rinne (16, 16') umfasst, wobei die Fortsätze (8) in den Rinnen (16, 16') angeordnet sind.

8. Zwischenwirbelfusionsimplantat nach einem der vorhergehenden Ansprüche,
**dadurch gekennzeichnet, dass**
der obere und untere Lagerkörper (2, 3) identisch ausgebildet sind.

9. Zwischenwirbelfusionsimplantat nach einem der Ansprüche 1 bis 7
**dadurch gekennzeichnet, dass**
der obere Lagerkörper 2 ein oberes Haltemodul 20 aufweist und der untere Lagerkörper 3 ein unteres Haltemodul 21.

10. Zwischenwirbelfusionsimplantat nach einem der vorhergehenden Ansprüche,
**dadurch gekennzeichnet, dass**
jeder Lagerkörper (2, 3) vier oder mehr Fortsätze (8) aufweist.

11. Zwischenwirbelfusionsimplantat nach einem der vorhergehenden Ansprüche,
**dadurch gekennzeichnet, dass**
die Lagerkörper (2, 3) Lagerflächen (4, 5) mit einer knochenwachstums-fördernden Beschichtung (19) aufweisen.

12. Zwischenwirbelfusionsimplantat nach einem der vorhergehenden Ansprüche,
**dadurch gekennzeichnet, dass**
das Zwischenwirbelfusionsimplantat (1) in der Koppelstellung eine Höhe zwischen 3 mm und 9 mm, vorzugsweise 6 mm, und in der Bewegungsstellung eine Höhe zwischen 9,5 mm und 20 mm, vorzugsweise 12,5 mm, und einen Durchmesser zwischen 12 mm und 30 mm aufweist, wobei der Sperrkörper (10) eine Stärke zwischen 0,5 mm und 3 mm aufweist.

13. Zwischenwirbelfusionsimplantat nach einem der vorhergehenden Ansprüche,
**dadurch gekennzeichnet, dass**
der Sperrkörper (10) aus einem gleitgünstigen zähfesten Kunststoff, insbesondere PEEK oder UHMWPE besteht.

14. Zwischenwirbelfusionsimplantat nach einem der vorhergehenden Ansprüche,
**dadurch gekennzeichnet, dass**
die Lagerkörper (2, 3) und die Klauenkupplung (9) aus einer metallischen biokompatiblen Legierung, insbesondere aus Reintitan, einer Titanlegierung, einer Kobaltbasislegierung CoCr oder CoCrMo; oder aus einem hochfesten biokompatiblen Kunststoff, insbesondere PEEK, PEEK-Kohlenstoff oder Keramik besteht.

15. System aus einem Zwischenwirbelfusionsimplantat (1) zur Fusion zweier Wirbel (52, 53) umfassend einen oberen Lagerkörper (2) zur Anlage an eine untere Endplatte eines Wirbels (52) und einen unteren Lagerkörper (3) zur Anlage an eine obere Endplatte eines gegenüberliegenden Wirbels (53), sowie ein zwischen den Lagerkörpern (2, 3) angeordnetes Mittelstück, und einer Zange (100) mit einem Maul (101), das ein erstes und ein zweites Backenelement (102, 103) aufweist,
**dadurch gekennzeichnet, dass**
das Mittelstück als eine Klauenkupplung (9) ausgebildet ist, die in einer Koppelstellung die beiden Lagerkörper (2, 3) drehfest gegeneinander koppelt, wobei in einer offenen Stellung der Klauenkupplung (9) die Lagerkörper (2, 3) gegeneinander frei beweglich sind, wobei die Klauenkupplung (9) automatisch betätigt ist und als Betätigungsorgan einen plastischen Sperrkörper (10) aufweist, der in seiner initialen Stellung die Klauenkupplung (9) offen hält und unter einer von dem einen Wirbel (52) auf den anderen Wirbel (54) übertragenen Lastkraft langsam verformbar ist bis zum Erreichen einer finalen Koppelstellung, in der die Klauenkupplung (9) geschlossen ist; und
die Backenelemente (102, 103) zur positionsversetzten Anordnung der am oberen und unteren Lagerkörper (2, 3) angeordneten Fortsätze (8) ausgebildet ist, so dass die Fortsätze (8) des oberen Lagerkörpers (2) neben den Fortsätzen (8) des unteren Lagerkörpers (3) angeordnet sind.

16. System nach Anspruch 15
**dadurch gekennzeichnet, dass**
die Zange (100) ein Anschlagselement (104) zur Einstellung eines Mindestabstands der Backenelemente (102, 103) aufweist, wobei die Backenelemente (102, 103) das Zwischenwirbelfusionsimplantat (1) in der offenen Stellung aufnehmen.

17. System nach Anspruch 15 oder 16,
**dadurch gekennzeichnet, dass**
das Zwischenwirbelfusionsimplantat (1) nach einem der Ansprüche 2 bis 14 ausgebildet ist.
